# Europäisches Patentamt
# European Patent Office
# Office européen des brevets

(19)

(11) Veröffentlichungsnummer: **0 040 431**
**B1**

(12) **EUROPÄISCHE PATENTSCHRIFT**

(45) Veröffentlichungstag der Patentschrift:
**02.11.83**

(21) Anmeldenummer: **81103850.4**

(22) Anmeldetag: **19.05.81**

(51) Int. Cl.³: **C 12 Q 1/34**, G 01 N 33/52

(54) **Verfahren und Reagens zur Bestimmung von beta-Lactamasen.**

(30) Priorität: **21.05.80 DE 3019451**

(43) Veröffentlichungstag der Anmeldung:
**25.11.81 Patentblatt 81/47**

(45) Bekanntmachung des Hinweises auf die Patenterteilung:
**02.11.83 Patentblatt 83/44**

(84) Benannte Vertragsstaaten:
**AT BE CH DE FR GB IT LI LU NL SE**

(56) Entgegenhaltungen:
**EP - A - 0 018 001**
**GB - A - 1 224 744**
**US - A - 3 644 177**
**US - A - 3 830 700**
**US - A - 4 164 448**
**US - A - 4 202 938**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Stahl, Peter, Dr., Hirtenstrasse 12, D-8131 Bernried (DE)**
Erfinder: **Vömel, Wolfgang, Dr., Am Aubuckel 26, D-6800 Mannheim 51 (DE)**
Erfinder: **Seidel, Hans, Dr., Waxensteinstrasse 6, D-8132 Tutzing (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren und Reagens zur Bestimmung von β-Lactamasen

Die Erfindung betrifft ein Verfahren und ein Reagens zur Bestimmung von β-Lactamasen unter Verwendung von 7-Cyanoacetylaminocephalosporansäure.

Penicilline und Cephalosporine als weit verbreitete Antibiotika sind chemisch durch das Vorliegen eines β-Lactamrings ausgezeichnet. Dieser β-Lactamring wird unter Inaktivierung dieser Antibiotika von einer Gruppe von Enzymen gespalten, die als β-Lactamasen bezeichnet werden. Auf der Bildung von β-Lactamasen beruht daher ein wichtiger Resistenzmechanismus von mikrobiellen grampositiven und gramnegativen Krankheitserregern. Bisher wurde angenommen, dass die β-Lactamaseaktivität von Krankheitserregern in die Resistenzbestimmung bzw. Bestimmung der minimalen Hemmkonzentration (MHK) mit eingeht. Nunmehr wurde festgestellt, dass eine derartige Korrelation nicht vorliegt. Ein gegenüber einem β-Lactam-Antibiotikum im Agartest als sensibel erkannter Krankheitserreger kann trotzdem beim Patienten infolge seiner zu einer späteren Wachstumsphase gebildeten β-Lactamaseaktivität resistent sein. Daher besteht ein Bedürfnis nach einem praktikablen Routinetest auf β-Lactamaseaktivität als Ergänzung der Resistenzbestimmung mit β-Lactamantibiotika. Ein derartiger Test sollte extrazelluläre und periplasmatische β-Lactamasen möglichst spezifisch nachweisen und über eine stabile, empfindliche Indikatorreaktion durchgeführt werden können.

Aus der US-A Nr. 3830700 sind Cephalosporinderivate bekannt, welche durch β-Lactamasen direkt unter Farbstoffbildung gesplaten werden. Diese Farbreaktion tritt in unspezifischer Weise jedoch auch bei Serumalbumin, Eiweiss, Cystein, Glutathion, u. a. ein. Diese Fehlerquelle macht die Verbindungen für die Praxis ungeeignet.

Handelsüblich ist ein β-Lactamase-Test, bei welchem aus Benzylpenicillin Penicillinsäure gebildet und letztere mit einem geeigneten pH-Indikator bestimmt wird. Nachteile dieser Methode liegen darin, dass sie nicht auf extrazelluläre β-Lactamasen anwendbar ist, der pH-Indikator unspezifisch ist, eine Übertragbarkeit auf die Cephalosporinresistenz weitgehend nicht besteht und für viele β-Lactamaseproduzenten die Testempfindlichkeit nicht ausreicht.

Bekannt ist weiter ein Verfahren zur Bestimmung von β-Lactamase durch Zusatz von 7-Cyanoacetylaminocephalosporansäure (als Natriumsalz), einem unter der Bezeichnung Cephacetril handelsüblichen Antibiotikum („Microbios Letters" [1976], 3, 35-39). An der Luft bildet sich hierbei ein roter Farbstoff, dessen Menge bestimmt werden kann. Dieses Verfahren ist jedoch für eine Routinebestimmung nicht brauchbar, da zur Farbentwicklung eine Inkubationszeit von 24 h bei 37° C erforderlich ist.

Nunmehr wurde gefunden, dass es möglich ist, durch bestimmte Zusätze diese Farbbildung so zu beschleunigen, dass der Test innerhalb einiger Minuten durchgeführt werden kann.

Dies wird erfindungsgemäss erreicht durch ein Verfahren zur Bestimmung von β-Lactamasen unter Zusatz von 7-Cyanoacetylaminocephalosporansäure und Messung des gebildeten Farbstoffs, welches dadurch gekennzeichnet ist, dass man Ammoniumionen zusammen mit Phosphationen oder/und ein Sauerstoff abspaltendes Mittel zusetzt.

Vorzugsweise werden erfindungsgemäss sowohl Ammoniumionen und Phosphationen als auch das Sauerstoff abspaltende Mittel zugegeben.

Weder Ammoniumionen noch Phosphationen allein üben eine beschleunigende Wirkung auf die Farbstoffbildung aus. Es ist daher überraschend, dass der Kombination dieser beiden Ionen eine synergistische Wirksamkeit zukommt, die zu einer sehr starken Beschleunigung der Farbstoffbildungsreaktion führt. Ebensowenig war zu erwarten, dass ein Sauerstoff abspaltendes Mittel die Geschwindigkeit wesentlich erhöhen würde, da ja Luftsauerstoff stets im Überschuss vorliegt.

Als Sauerstoff abspaltendes Mittel wird vorzugsweise Katalase zusammen mit $H_2O_2$ verwendet. $H_2O_2$ kann als solches, d. h. in flüssiger Form, in Form eines Addukts, welches zumeist in fester Form existent ist, oder in Form eines $H_2O_2$ liefernden Systems zugesetzt werden. Als $H_2O_2$ lieferndes Mittel bzw. System kommt im Rahmen der Erfindung eine Oxidase zusammen mit ihrem reduzierten Substrat in Betracht. Als Beispiele hierfür seien genannt Glucoseoxidase und Glucose, Cholesterinoxidase und Cholesterin, Xanthinoxidase und Xanthin, Diaminoxidase und ein Amin wie Hexamethylendiamin, Histamin usw. Beispiele für $H_2O_2$-Addukte sind Harnstoffperoxohydrat, ein Phosphatperoxohydrat, Boratperoxohydrat oder Carbonatperoxohydrat, zweckmässig in Form der Ammoniumsalze. Infolge des Gehalts an Ammoniumionen, Phosphationen und $H_2O_2$ ist Ammoniumperoxophosphat besonders interessant.

Ammoniumionen werden in Form eines entsprechenden Salzes, beispielsweise als Phosphat, Chlorid oder Sulfat, zugesetzt. Die Phosphationen werden zweckmässig in Form von Alkalisalzen verwendet, wobei diese vorzugsweise gleichzeitig zur pH-Werteinstellung dienen. Für die Reaktion selbst ist auf einen pH-Wert abzustellen, bei welchem die zu bestimmenden β-Lactamasen aktiv sind. Im allgemeinen gut geeignet sind pH-Werte zwischen 5,5 und 8,5. Bevorzugt wird der Bereich von 6,5 bis 7.

Das erfindungsgemässe Verfahren kann bei Zimmertemperatur oder bei erhöhter Temperatur durchgeführt werden. Da die β-Lactamasen relativ temperaturstabil sind, wird vorzugsweise bei erhöhter Temperatur zwischen 30 und 65° C gearbeitet.

Das erfindungsgemässe Verfahren kann in üblicher Weise als Küvettentest durchgeführt werden, und zwar sowohl als Endpunktmethode als auch kinetisch. Bei einer Reaktionstemperatur von 60° C und Zugabe von Ammoniumionen zusammen mit Phosphationen und $H_2O_2$ ist unter diesen Bedingungen der Reaktionsendpunkt nach 5 min erreicht, bei 37° C wird der Endpunkt nach 10 bis 15 min erhalten. Bei dieser Temperatur ist daher ein kinetisches Verfahren vorzuziehen. Das erfindungsgemässe Verfahren eignet sich aber auch für den Mikrotitertest, Streifentest oder Plattentest. Im Falle des Mikrotiter-, Streifen- oder Platten-tests wird vor allem bei gramnegativen Keimen durch einen Komplexbildner, wie EDTA und/oder einen Detergenszusatz die Empfindlichkeit gesteigert. Es wird angenommen, dass durch das Detergens und/oder den Komplexbildner die Permeabilität der gramnegativen Zellen erhöht wird.

Gemäss einer besonders zweckmässigen und bevorzugten Ausführungsform des erfindungsgemässen Verfahrens trägt man zur Durchführung auf einer Kulturplatte, insbesondere auf einer angeimpften bzw. einer vorinkubierten Kulturplatte, die für die Umsetzung erforderlichen Reagenzien auf eine Stelle der Kulturoberfläche auf und bringt um diese Stelle herum eine für den gebildeten Farbstoff undurchlässige Sperrschicht ein. Dies kann zweckmässigerweise darin bestehen, dass man ein ringförmiges Sperrglied aus Kunststoff, Glas oder Metall, beispielsweise einen Ring entsprechenden Durchmessers, um die Auftragsstelle in die Kultur einsetzt, beispielsweise eindrückt. Dadurch wird ein Ausdiffundieren des gebildeten Farbstoffs in die Platte verhindert und die Genauigkeit der Messung des Farbstoffs erhöht. Die Messung selbst kann visuell, z. B. durch Vergleich mit einer Farbskala, oder durch Bestimmung der Absorption zwischen etwa 400 und 600 nm gemessen werden.

Die erfindungsgemäss erzielbare Beschleunigung der Farbstoffbildung tritt in einem sehr weiten Bereich der Konzentration von Phosphationen, Ammoniumionen bzw. $H_2O_2$ auf. Zweckmässig sollte die Konzentration bei Phosphationen 0,001 bis 1 mol/l, bei Ammoniumionen 0,01 bis 3 mol/l und bei $H_2O_2$ bis 0,5 mol/l betragen. Die besten Ergebnisse werden erzielt bei einer Phosphationenkonzentration von 0,1 bis 1 mol/l, einer Ammoniumionenkonzentration von 0,2 bis 1 mol/l und einer $H_2O_2$-Konzentration von 0,05 bis 0,2 mol/l.

Ein weiterer Gegenstand der Erfindung ist ein Reagens zur Bestimmung von β-Lactam auf Basis von 7-Cyanoacetylaminocephalosporansäure, welches dadurch gekennzeichnet ist, dass es Ammoniumionen in Kombination mit Phosphationen oder/und ein Sauerstoff abspaltendes Mittel enthält.

In einer bevorzugten Ausführungsform enthält ein derartiges Reagens 0,1 bis 100 mg/ml 7-Cyanoacetylaminocephalosporansäure, 0,01 bis 0,5 mol $H_2O_2$/l, 0,001 bis 0,5 mol Phosphationen/l, 0,01 bis 3 mol Ammoniumionen/l sowie 5 bis 1000 U Katalase pro Test und weist einen pH-Wert von 5,5 bis 8,5 auf, und 2,5 bis 100 mmol/ml EDTA.

Das Reagens kann als trockene Mischung der festen Substanzen vorliegen, die vor Gebrauch mit Wasser auf die angegebene Konzentration aufgefüllt wird, oder kann in gelöster Form vorliegen.

Gemäss einer besonderen Ausführungsform des erfindungsgemässen Reagens liegt es in imprägnierter Form auf einem saugfähigen inerten Träger vor. Als Träger kommen beispielsweise in Betracht Papierstreifen oder -plättchen, saugfähige Kunststoffolien, granuliertes saugfähiges Material oder dergleichen. Diese Ausführungsform eignet sich besonders beim Plattentest. Es genügt dann, ein entsprechendes imprägniertes Plättchen auf die zu untersuchende Kulturstelle aufzulegen, mit Wasser zu befeuchten und die gebildete Färbung zu bestimmen. Vorzugsweise wird hierbei die oben bereits erwähnte Ausführungsform mit Anbringung einer undurchlässigen Sperrschicht angewendet. Es wird also ein Ring aus Kunststoff, Metall oder Glas mit einem dem Plättchen entsprechenden oder etwas grösseren Durchmesser über das Plättchen gelegt und durch die Kulturplatte hindurchgedrückt, so dass die gebildete Farbe innerhalb dieses Ringes konzentriert bleibt.

Vorzugsweise kann dieses System als Ergänzung zur Resistenzbestimmung dienen, da ein gegenüber einem β-Lactam-Antibiotikum im Agardiffusionstests als sensibel erkannter Krankheitserreger trotzdem infolge seiner zu einer späteren Wachstumsphase gebildeten β-Lactamaseaktivität beim Patienten resistent sein kann. Zu diesem Zweck werden auf bereits angewachsenen Keimausstrichen Testplättchen und Sperrschicht (z. B. Kunststoffzylinder) aufgebracht und die Testplatte weiter inkubiert. Die Rotverfärbung der Testplättchen zeigt die Bildung extrazellulärer oder periplasmatischer β-Lactamaseproduktion während des Keimwachstums an.

Das erfindungsgemässe Verfahren und Reagens lässt sich zur Bestimmung aller β-Lactamasen (Penicillinase/Cephalosporinase) anwenden. Als Beispiele seien genannt β-Lactamase I und II aus *Bacillus cereus*, TEM-β-Lactamase aus *E. coli*, β-Lactamase aus *Enterobacter cloacae*, aus *Staphilococcus aureus* und *Aerobacter aerogenes*.

Mit dem erfindungsgemässen Verfahren lassen sich β-Lactamasekonzentrationen bis herab zu 0,05 U/ml nachweisen. Aufgrund dieser hohen Empfindlichkeit kann das Verfahren auch zu einem generellen Keimnachweis insbesondere für gramnegative Bakterien eingesetzt werden, beispielsweise in Urin.

Die folgenden Beispiele erläutern die Erfindung weiter.

### Beispiel 1

In eine Küvette werden 26 U Katalase (10 µl eines 1:100 verdünnten handelsüblichen Präparats), 20 µl 3,3%ige $H_2O_2$, 2 ml 0,1 mol/l Phosphatpuffer, pH 6,5 und 200 µl gesättigte Ammoniumsulfatlösung sowie 100 µl 7-Cyanoacetylaminocephalosporansäurelösung (C = 100) einpipettiert und auf 60° C thermostatiert. Dann werden 170 µl lactamasehaltige Testlösung zugesetzt. Nach 10 min wird die Extinktion bei 546 nm bestimmt.

*Beispiel 2*

*Mikrotitertest*

10 µl Katalaselösung (26 U), 20 µl 3,3%ige $H_2O_2$-Lösung, 100 µl 0,1 mol/l Phosphatpuffer, pH 7, 20 µl gesättigte Ammonsulfatlösung und 10 µl 7-Cyanoacetylaminocephalosporansäurelösung (c = 100) werden mit 50 µl Probelösung versetzt und 5 min auf 60°C erwärmt. Die gebildete Rotfärbung wird anhand einer Vergleichsskala halbquantitativ bestimmt.

*Beispiel 3*

10 µl Katalaselösung (26 U), 20 µl 3,3%ige $H_2O_2$-Lösung, 10 µl 1 mol/l Phosphatpuffer, pH 7, 20 µl gesättigte Ammonsulfatlösung und 10 µl 7-Cyanoacetylaminocephalosporansäurelösung (c = 100) werden mit 200 µl Flüssigkultur versetzt und 5 min auf 60°C erwärmt. Die gebildete Rotfärbung wird anhand einer Vergleichsskala halbquantitativ bestimmt.

*Beispiel 4*

Filterpapier, insbesondere Filterblättchen mit 6 bis 9 mm Durchmesser, wird mit einer Lösung imprägniert, welche 100 U Katalase/ml, 1 mol Harnstoffperhydrat/l, 0,5 mol Ammonsulfat/l in 0,1M Phosphatpuffer, pH 6,5, 1 g/l 7-Cyanoacetylaminocephalosporansäure und 1 g/l eines nichtionischen Detergens bzw. 25 mmol EDTA enthielt. Das imprägnierte Filterpapier wurde getrocknet und in Streifen von 1 cm Breite geschnitten.

So erhaltene Teststreifen/Plättchen wurden auf die Oberfläche einer beimpften Agarkulturplatte gelegt. Ein Kunststoffring von 1,5 cm bzw. 0,7 bis 1,0 cm Durchmesser und 10 mm Höhe wurde über das Plättchen auf die Agarplatte gestellt und in das Agar hineingedrückt. Nach 5 min Entwicklung bei 60°C wurde das nunmehr rot gefärbte Plättchen entnommen und die Farbstärke mit Hilfe einer Vergleichsskala bestimmt.

**Patentansprüche**

1. Verfahren zur Bestimmung von β-Lactamase durch Zusatz von 7-Cyanoacetylaminocephalosporansäure und Messung des dabei gebildeten Farbstoffs, dadurch gekennzeichnet, dass man Ammoniumionen in Kombination mit Phosphationen oder/und ein Sauerstoff absplatendes Mittel zusetzt.

2. Verfahren nach Anspruch 1, dadurch gekennzeichnet, dass man als Sauerstoff abspaltendes Mittel Katalase zusammen mit $H_2O_2$ oder einem $H_2O_2$ liefernden Mittel zusetzt.

3. Verfahren nach Anspruch 2, dadurch gekennzeichnet, dass das $H_2O_2$ liefernde Mittel aus einer Oxidase und ihrem reduzierten Substrat besteht.

4. Verfahren nach Anspruch 2, dadurch gekennzeichnet, dass das $H_2O_2$ liefernde Mittel aus einem $H_2O_2$-Addukt besteht.

5. Verfahren nach Anspruch 4, dadurch gekennzeichnet, dass man als $H_2O_2$-Addukt Harnstoffperoxohydrat oder ein Phosphatperoxohydrat verwendet.

6. Verfahren nach einem der vorhergehenden Ansprüche, dadurch gekennzeichnet, dass man die Umsetzung bei erhöhter Temperatur ausführt.

7. Verfahren nach einem der vorhergehenden Ansprüche, dadurch gekennzeichnet, dass man zur Durchführung der Bestimmung auf einer Kulturplatte die Reagenzien auf einer Stelle der Kulturoberfläche aufträgt und um diese Stelle herum eine für den gebildeten Farbstoff undurchlässige Sperrschicht in die Kultur einbringt.

8. Verfahren nach Anspruch 7, dadurch gekennzeichnet, dass man ein ringförmiges Sperrglied aus Kunststoff oder Metall um die Auftragsstelle in die Kultur einsetzt.

9. Verfahren nach einem der vorhergehenden Ansprüche, dadurch gekennzeichnet, dass man Phosphationen bis zu einer Molarität von 0,001 bis 1 mol/l, Ammoniumionen bis zu einer Molarität von 0,01 bis 3 mol/l oder/und $H_2O_2$ bis zu einer Molarität von 0,01 bis 0,5 mol/l zusetzt.

10. Reagens zur Bestimmung von β-Lactamasen auf Basis von 7-Cyanoacetylaminocephalosporansäure, dadurch gekennzeichnet, dass es Ammoniumionen in Kombination mit Phosphationen oder/und ein Sauerstoff abspaltendes Mittel enthält.

11. Reagens nach Anspruch 10, dadurch gekennzeichnet, dass es 0,1 bis 100 mg/ml 7-Cyanoacetylaminocephalosporansäure, 0,01 bis 0,5 mol $H_2O_2$/l, 0,001 bis 0,05 mol Phosphationen/l, 0,01 bis 3 mol Ammoniumionen/l, 5 bis 1000 U Katalase/Testvolumen enthält und einen pH-Wert von 5,5 bis 8,5 aufweist und 2,5 bis 100 mmol/l EDTA bzw. Detergens.

12. Reagens nach einem der beiden Ansprüche 10 oder 11, dadurch gekennzeichnet, dass es auf einem festen saugfähigen Trägermaterial imprägniert vorliegt.

13. Reagens nach Anspruch 10, 11 oder 12, dadurch gekennzeichnet, dass es Substanzen enthält, welche die äussere Bakterienmembran durchlässiger machen.

14. Reagens nach Anspruch 13, dadurch gekennzeichnet, dass es als durchlässig machende Substanz ein Detergens, einen Chelatbildner (EDTA) oder/und ein Antibiotikum enthält.

**Revendications**

1. Procédé de détermination de β-Lactamase par addition d'acide 7-cyanoacétylaminocéphalosporanique et de mesure du colorant ainsi formé, caractérisé en ce qu'on ajoute des ions amonium en combinaison avec des ions phosphate ou/et un agent libérant de l'oxygène.

2. Procédé suivant la revendication 1, caractérisé en ce qu'on ajoute comme agent libérant de l'oxygène de la catalase en même temps que $H_2O_2$ ou qu'un agent fournissant $H_2O_1$.

3. Procédé suivant la revendication 2, caractérisé en ce que l'agent fournissant $H_2O_2$ se compose d'une oxydase et de son substrat réduit.

4. Procédé suivant la revendication 2, caractérisé en ce que l'agent fournissant $H_2O_2$ se compose d'un produit d'addition de $H_2O_2$.

5. Procédé suivant la revendication 4, caractérisé en ce qu'on utilise comme produit d'addition de $H_2O_2$ du peroxohydrate d'urée ou un peroxohydrate de phosphate.

6. Procédé suivant l'une des revendications précédentes, caractérisé en ce qu'on effectue la réaction à température augmentée.

7. Procédé suivant l'une des revendications précédentes, caractérisé en ce que, pour l'exécution de la détermination sur une plaque de culture, on dépose le réactif en un endroit de la surface de la culture et on dispose dans la culture autour de cet endroit une couche barrière imperméable au colorant formé.

8. Procédé suivant la revendication 7, caractérisé en ce qu'on enfonce un élément barrière en forme d'anneau en plastique ou en métal dans la culture autour de l'endroit du dépôt.

9. Procédé suivant l'une des revendications précédentes, caractérisé en ce qu'on ajoute des ions phosphate jusqu'à une molarité de 0,001 à 1 mol/l, des ions amonium jusqu'à une molarité de 0,01 à 3 mol/l ou/et $H_2O_2$ jusqu'à une molarité de 0,01 à 0,5 mol/l.

10. Réactif pour la détermination de β-Lactamases à base d'acide 7-cyanoacétylaminocéphalosporanique, caractérisé en ce qu'il contient des ions ammonium en combinaison avec des ions phosphate ou/et un agent libérant de l'oxygène.

11. Réactif suivant la revendication 10, caractérisé en ce qu'il contient: 0,1 à 100 mg/ml d'acide 7-cyanoacétylaminocéphalosporanique, 0,01 à 0,5 mol de $H_2O_2$ par litre, 0,01 à 0,5 mol d'ions phosphate par litre, 0,01 à 3 mol d'ions ammonium par litre, 5 à 1000 U de catalase par volume d'essai et présente une valeur de pH de 5,5 à 8,5 et 2,5 à 100 mmol/l d'EDTA ou de détergent.

12. Réactif suivant l'une des revendications 10 ou 11, caractérisé en ce qu'il se présente à l'état imprégné sur une matière de support absorbante solide.

13. Réactif suivant les revendications 10, 11 ou 12, caractérisé en ce qu'il contient une substance qui rend la membrane bactérienne externe plus perméable.

14. Réactif suivant la revendication 13, caractérisé en ce qu'il contient comme substance perméabilisante un détergent, un chélatant (EDTA) ou/et un antibiotique.

## Claims

1. Process for the determination of β-Lactamase by the addition of 7-cyanoacetylamino-cephalosporanic acid and measurement of the dyestuff thereby formed, characterised in that one adds ammonium ions in combination with phosphate ions and/or an agent splitting off oxygen.

2. Process according to claim 1, characterised in that, as agent splitting off oxygen, one adds catalase together with $H_2O_2$ or an $H_2O_2$-supplying agent.

3. Process according to claim 2, characterised in that the $H_2O_2$-supplying agent consists of an oxidase and its reducing substrate.

4. Process according to claim 2, characterised in that the $H_2O_2$-supplying agent consists of an $H_2O_2$ adduct.

5. Process according to claim 4, characterised in that, as $H_2O_2$ adduct, one uses urea peroxyhydrate or a phosphate peroxyhydrate.

6. Process according to one of the preceding claims, characterised in that one carries out the reaction at an elevated temperature.

7. Process according to one of the preceding claims, characterised in that for the carrying out of the determination on a culture plate, one applies the reagents to a place on the culture surface and provides around this place a barrier layer in the culture which is impermeable for the dyestuff formed.

8. Process according to claim 7, characterised in that one provides an annular barrier member of synthetic resin or metal around the point of application in the culture.

9. Process according to one of the preceding claims, characterised in that one adds phosphate ions up to a molarity of 0.001 to 1 mol/l, ammonium ions up to a molarity of 0.01 to 3 mol/l and/or $H_2O_2$ up to a molarity of 0.01 to 0.5 mol/l.

10. Reagent for the determination of β-Lactamase based upon 7-cyanoacetylaminocephalosporanic acid, characterised in that it contains ammonium ions in combination with phosphate ions and/or an agent splitting off oxygen.

11. Reagent according to claim 10, characterised in that it contains 0.1 to 100 mg/ml 7-cyanoacetylaminocephalosporanic acid, 0.01 to 0.5 mol $H_2O_2$ per liter, 0.001 to 0.5 mol phosphate ions per liter, 0.01 to 3 mol ammonium ions per liter, 5 to 1000 U catalase per test volume and has a pH value of 5.5 to 8.5 and 2.5 to 100 mmol/l EDTA or detergent.

12. Reagent according to claim 10 or 11, characterised in that it is present impregnated on a solid, absorbent carrier material.

13. Reagent according to claims 10, 11 or 12, characterised in that it contains a substance which makes the outer bacterial membrane more permeable.

14. Reagent according to claim 13, characterised in that, as permeable-making substance, it contains a detergent, a chelate former (EDTA) and/or an antibiotic.